# EUROPEAN PATENT SPECIFICATION

(11) **EP 1 667 975 B1**
(45) Date of publication and mention of the grant of the patent: **28.11.2007**
(21) Application number: 04765655.8
(22) Date of filing: 23.09.2004
(51) Int. Cl.: C07D 215/40, A61K 31/47, A61P 25/00

(54) **A POLYMORPHIC FORM OF 3-PHENYLSULFONYL-8-PIPERAZIN-1-YL-QUINOLINE**
POLYMORPHE FORM VON 3-PHENYLSULFONYL-8-PIPERAZIN-1-YL-CHINOLIN
FORME POLYMORPHE DE QUINOLINE 3-PHENYLSULFONYL-8-PIPERAZIN-1-YL

(30) Priority: 26.09.2003 GB 0322629
(43) Date of publication of application: 14.06.2006
(73) Proprietor: GLAXO GROUP LIMITED, Greenford, Middlesex UB6 ONN (GB)
(72) Inventor: GLADWIN, Asa, Elisabeth GlaxoSmithKline, Stevenage, Hertfordshire SG1 2NY (GB)
(74) Representative: Knight, Lucie Viktoria
(86) International application number: PCT/EP2004/010843
(87) International publication number: WO 2005/040124

(56) References cited:
- WO-A-03/080580

## Description

This invention relates to a novel compound having pharmacological activity, to processes for its preparation, to compositions containing it and to its use in the treatment of CNS and other disorders.

WO 2003/080580 (Glaxo Group Limited) describes the preparation of 3-phenylsulfonyl-8-piperazin-1-yl-quinoline (Example 16) in addition to two polymorphic forms of 3-phenylsulfonyl-8-piperazin-1-yl-quinoline (Form I; Example 51 and Form II; Example 52). 3-Phenylsulfonyl-8-piperazin-1-yl-quinoline is disclosed in WO 2003/080580 as having affinity for the 5-HT₆ receptor and is claimed to be useful in the treatment of CNS and other disorders.

It has now been found that 3-phenylsulfonyl-8-piperazin-1-yl-quinoline exists in a further polymorphic form which is primarily characterised in that it possesses a higher melting point than Forms I and II. This further polymorphic form of 3-phenylsulfonyl-8-piperazin-1-yl-quinoline is referred to as Form III.

Thus, according to a first aspect of the present invention, we provide 3-phenylsulfonyl-8-piperazin-1-yl-quinoline Form III.

Suitably, the present invention provides 3-phenylsulfonyl-8-piperazin-1-yl-quinoline, suitably as characterised by data provided by at least one of the following: infrared, Raman, X-ray powder diffraction or nuclear magnetic resonance and melting point data as provided herein, including partial spectral data provided herein.

Thus, one aspect of the present invention provides a polymorphic form of 3-phenylsulfonyl-8-piperazin-1-yl-quinoline, characterised in that it provides:
(i) an infrared spectrum containing peaks at 724, 758, 777, 804, 818, 838, 856, 905, 918, 948, 1023, 1055, 1081, 1092, 1118, 1136, 1153, 1178, 1244, 1302, 1318, 1365, 1378, 1403, 1444, 1471, 1490, 1569, 1584, 1603 and 2819 cm⁻¹; and/or
(ii) a Raman spectrum containing peaks at 159, 184, 214, 241, 285, 304, 318, 429, 545, 558, 614, 706, 724, 803, 856, 1000, 1023, 1080, 1093, 1136, 1152, 1233, 1243, 1317, 1343, 1364, 1378, 1403, 1446, 1569, 1584, 1602, 3050 and 3073 cm⁻¹; and/or
(iii) an X-ray powder diffraction (XRPD) pattern which gives calculated lattice spacings at 10.29, 11.94, 17.47, 19.55, 19.84, and 20.33°; and/or
(iv) a melting point of 188°C.

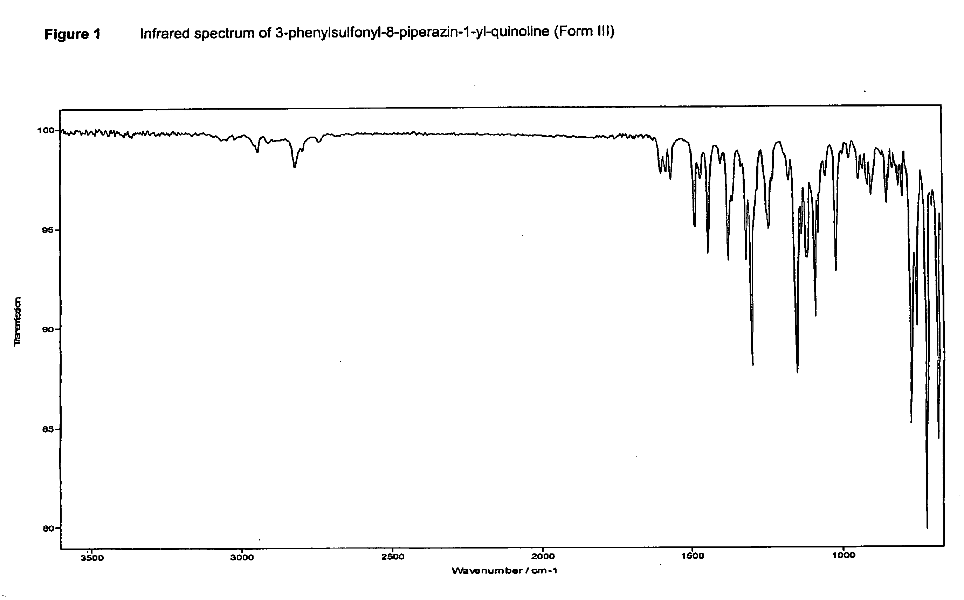

In one preferred aspect, the polymorph of the present invention provides an infrared spectrum substantially in accordance with Figure 1.

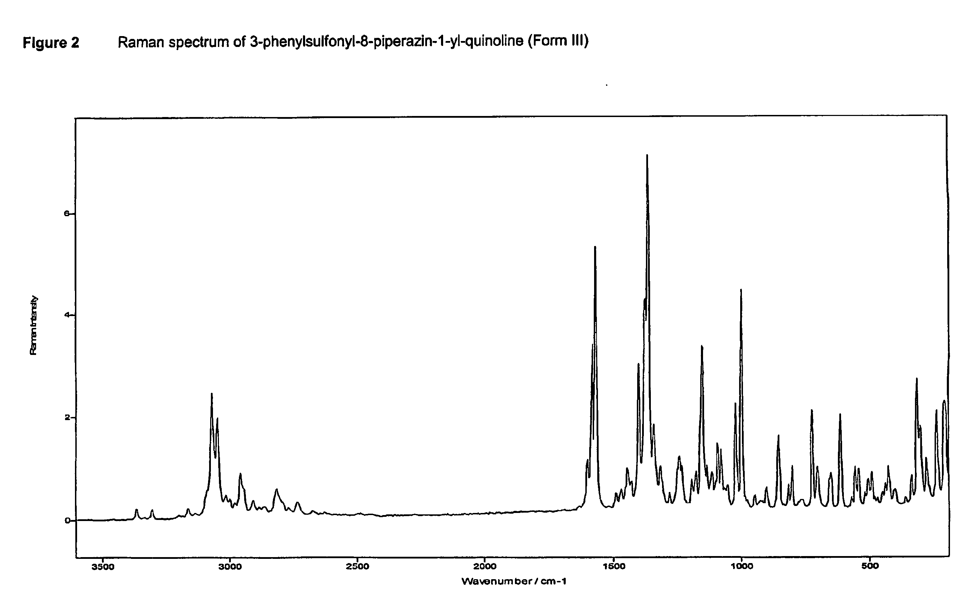

In one preferred aspect, the polymorph of the present invention provides a Raman spectrum substantially in accordance with Figure 2.

In one preferred aspect, the polymorph of the present invention provides an X-ray powder diffraction (XRPD) pattern which gives calculated lattice spacings at 10.29, 10.76, 11.94, 14.33, 14.61, 14.93, 16.02, 16.80, 17.47, 17.92, 19.13, 19.55, 19.84, 20.33, 21.16, 21.36, 23.33, 23.96, 24.44, 24.67, 25.51, 26.12, 27.13, 27.77, 28.06, 28.35, 29.23, 29.46, 30.06, 30.35, 31.27, 32.35, 32.66, 33.08, 33.77, 34.49, 35.18, 36.42, 37.34, 38.39 and 39.51°.

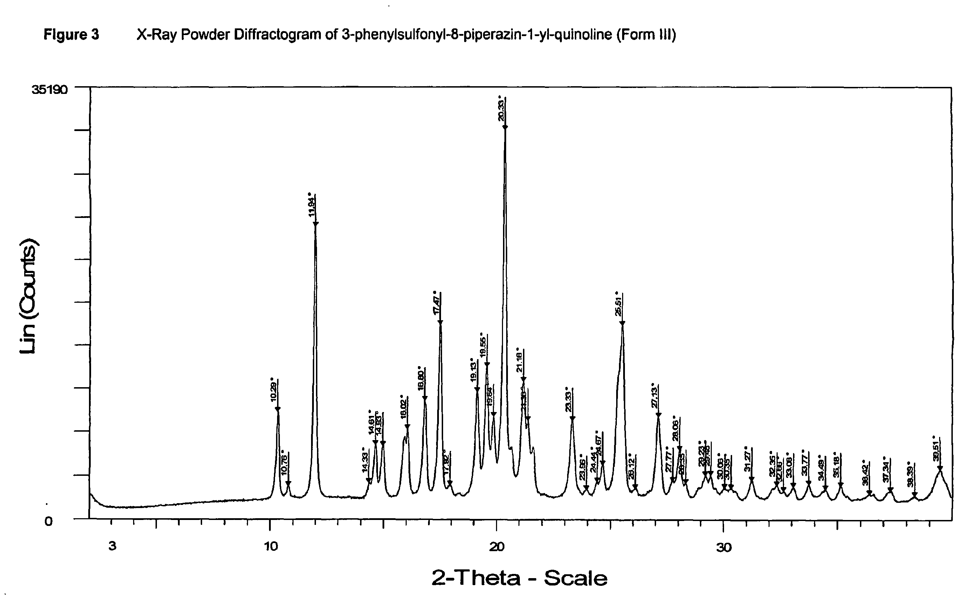

In a more preferred aspect, the polymorph of the present invention provides an X-ray powder diffraction (XRPD) pattern substantially in accordance with Figure 3.

The present invention encompasses the polymorph isolated in pure form or when admixed with other materials, for example the known forms of 3-phenylsulfonyl-8-piperazin-1-yl-quinoline or any other material.

Thus, in one aspect there is provided the polymorph in isolated form. In a further aspect there is provided the polymorph in pure form. In yet a further aspect there is provided the polymorph in crystalline form. Most preferably, the polymorph is provided in pure form.

'Isolated in pure form' refers to 3-phenylsulfonyl-8-piperazin-1-yl-quinoline Form III present in an amount of preferably >75%, more preferably >90%, particularly >95%, especially >99% relative to other compounds or polymorphs of 3-phenylsulfonyl-8-piperazin-1-yl-quinoline which may be present in an isolated sample.

The compound of the present invention has affinity for the 5-HT₆ receptor and is believed to be of potential use in the treatment of certain CNS disorders such as anxiety, depression, epilepsy, obsessive compulsive disorders, migraine, cognitive memory disorders (e.g. Alzheimers disease, age related cognitive decline and mild cognitive impairment), Parkinsons Disease, ADHD (Attention Deficit Disorder/Hyperactivity Syndrome), sleep disorders (including disturbances of Circadian rhythm), feeding disorders such as anorexia and bulimia, panic attacks, withdrawal from drug abuse such as cocaine, ethanol, nicotine and benzodiazepines, schizophrenia (in particular cognitive deficits of schizophrenia), stroke and also disorders associated with spinal trauma and/or head injury such as hydrocephalus. The compound of the invention is also expected to be of use in the treatment of certain GI (gastrointestinal) disorders such as IBS (Irritable Bowel Syndrome). The compound of the invention is also expected to be of use in the treatment of obesity.

Thus the invention also provides 3-phenylsulfonyl-8-piperazin-1-yl-quinoline (Form III), for use as a therapeutic substance, in particular in the treatment or prophylaxis of the above disorders. In particular the invention provides for 3-phenylsulfonyl-8-piperazin-1-yl-quinoline (Form III), for use in the treatment of depression, anxiety, Alzheimers disease, age related cognitive decline, ADHD, obesity, mild cognitive impairment, schizophrenia, cognitive deficits in schizophrenia and stroke.

In another aspect, the invention provides the use of 3-phenylsulfonyl-8-piperazin-1-yl-quinoline (Form III) in the manufacture of a medicament for use in the treatment or prophylaxis of the above disorders.

5-HT₆ antagonists have the potential to be capable of increasing basal and learning-induced polysialylated neuron cell frequency in brain regions such as the rat medial temporal lobe and associated hippocampus, as described in WO 2003/066056.

In order to use the compound of the present invention in therapy, it will normally be formulated into a pharmaceutical composition in accordance with standard pharmaceutical practice. The present invention also provides a pharmaceutical composition, which comprises 3-phenylsulfonyl-8-piperazin-1-yl-quinoline (Form III), and a pharmaceutically acceptable carrier.

A pharmaceutical composition of the invention, which may be prepared by admixture, suitably at ambient temperature and atmospheric pressure, is usually adapted for oral, parenteral or rectal administration and, as such, may be in the form of tablets, capsules, oral liquid preparations, powders, granules, lozenges, reconstitutable powders, injectable or infusable solutions or suspensions or suppositories. Orally administrable compositions are generally preferred.

Tablets and capsules for oral administration may be in unit dose form, and may contain conventional excipients, such as binding agents, fillers, tabletting lubricants, disintegrants and acceptable wetting agents. The tablets may be coated according to methods well known in normal pharmaceutical practice.

Oral liquid preparations may be in the form of, for example, aqueous or oily suspension, solutions, emulsions, syrups or elixirs, or may be in the form of a dry product for reconstitution with water or other suitable vehicle before use. Such liquid preparations may contain conventional additives such as suspending agents, emulsifying agents, non-aqueous vehicles (which may include edible oils), preservatives, and, if desired, conventional flavourings or colourants.

For parenteral administration, fluid unit dosage forms are prepared utilising a compound of the invention or pharmaceutically acceptable salt thereof and a sterile vehicle. The compound, depending on the vehicle and concentration used, can be either suspended or dissolved in the vehicle. In preparing solutions, the compound can be dissolved for injection and filter sterilised before filling into a suitable vial or ampoule and sealing. Advantageously, adjuvants such as a local anaesthetic, preservatives and buffering agents are dissolved in the vehicle. To enhance the stability, the composition can be frozen after filling into the vial and the water removed under vacuum. Parenteral suspensions are prepared in substantially the same manner, except that the compound is suspended in the vehicle instead of being dissolved, and sterilization cannot be accomplished by filtration. The compound can be sterilised by exposure to ethylene oxide before suspension in a sterile vehicle. Advantageously, a surfactant or wetting agent is included in the composition to facilitate uniform distribution of the compound.

The composition may contain from 0.1 % to 99% by weight, preferably from 10 to 60% by weight, of the active material, depending on the method of administration.

The dose of the compound used in the treatment of the aforementioned disorders will vary in the usual way with the seriousness of the disorders, the weight of the sufferer, and other similar factors. However, as a general guide suitable unit doses may be 0.05 to 1000 mg, more suitably 0.05 to 200 mg, for example 20 to 40 mg; and such unit doses will preferably be administered once a day, although administration more than once a day may be required; and such therapy may extend for a number of weeks or months.

The following Descriptions and Examples illustrates the preparation of the compound of the invention.

### Description 1

### 3-Iodo-8-nitroquinoline (D1)

A stirred mixture of 8-nitroquinoline (100 g, 0.57 mol) in acetic acid (500 ml) was treated with *N*-iodosuccinimide (155 g, 0.69 mol) portionwise over 10 minutes, and warmed to 62°C for 6 h. A further portion of *N*-iodosuccinimide (25 g, 0.14 mol) was introduced and the mixture stirred for a further 16 h before cooling to ambient temperature. The solvent was removed *in vacuo,* keeping the temperature below 35°C. The residue was dissolved in dichloromethane (2 L) and washed successively with saturated aqueous sodium bicarbonate solution (2 x 1 L), 10% aqueous sodium thiosulphate solution (1L), water (1 L), brine (100 ml), then the organic phase was dried over magnesium sulphate. The mixture was filtered and the solvent removed to give a yellow solid which was recrystallised from ethyl acetate to give the title compound (D1) (168 g, 97%) as a yellow solid;
δ_{H} (CDCl₃) 7.65 (1H, app.t), 7.94 (1H, dd), 8.07 (1H, dd), 8.66 (1H, d, J = 2Hz), 9.19 (1H, d, J = 2Hz);
Mass Spectrum : C₉H₅IN₂ requires 300; found 301 (MH⁺).

### Description 2

### 8-Nitro-3-phenylsulfonylquinoline (D2)

3-lodo-8-nitroquinoline (D1) (135 g, 0.45 mol), was suspended in dimethylformamide (2.4 L) in a 5 L 3-necked flask fitted with an overhead stirrer, under an argon atmosphere. This mixture was treated successively with anhydrous sodium phenylsulfinate (99.6 g 0.608 mol), and bis-(copper (I) triflate) benzene complex (170 g, 0.338 mol). The resulting slurry was heated to 65 °C for 18 h. The mixture was cooled, filtered and the solvent evaporated *in vacuo.* Acetone (2.5 L) was added to the residue and the solution filtered. The filtrate was evaporated *in vacuo,* a further 2.5 L of acetone added and the mixture filtered again. The solvent was evaporated *in vacuo* and the residue dissolved in chloroform (3 L) and washed with 10% aqueous ammonia (2 x 2 L), and the organic phase was dried over magnesium sulphate and the solvent evaporated *in vacuo.* The dark brown residue was purified using a Biotage flash-150 chromatography apparatus (5 kg silica gel) eluting with hexane and increasing proportions of ethyl acetate to give the title compound (D2) (81.5 g, 58%) as a yellow solid;
δ_{H} (d6-DMSO) 7.67 (2H, t), 7.57 (1H, d, 7.96 (1H, t), 8.13 (2H, d), 8.51 (1H, d), 8.59 (1H, d), 9.42 (1H, d), 9.50 (1H, d);
Mass Spectrum : C₁₅H₁₀SO₄N₂ requires 314; found 315 (MH⁺).

### Description 3

### 8-Amino-3-phenylsulfonylquinoline (D3)

A slurry of 8-nitro-3-phenylsulfonylquinoline (D2) (46.7 g, 172 mmol), in tetrahydrofuran (750 ml) was added to a stirred solution of 30% titanium (III) chloride in aqueous HCl (470 ml) [Supplied by BDH] cooled in an ice bath, at such a rate that the temperature was maintained below 35°C. Once the addition was completed, the solution was stirred for a further 10 minutes then water (1.5 L) was introduced and the mixture poured into a 5 L beaker. The rapidly stirred solution was treated by portionwise addition of solid potassium carbonate in order to attain pH -8.5. EDTA (250 g, 0.86 mol) was added and followed by further potassium carbonate to maintain pH -8.5. The mixture was extracted with dichloromethane (3 x 1L) and the combined organic phase passed through a silica plug (500 g) eluting with further dichloromethane (1 L) and 10% ethyl acetate in dichloromethane (1 L). The combined organic phases were evaporated and the residue subjected to purification using Biotage Flash-75 chromatography apparatus (2 kg silica gel), eluting with dichloromethane and increasing proportions of ether to give the title compound (D3) (34.5 g, 72%) as a pale brown solid;
δ_{H} (CDCl₃) 5.0 (2H, br s), 7.02 (1H, dd), 7.25 (1H, dd), 7.44 (1H, t), 7.50-7.59 (3H, m), 8.00-8.40 (2H, m), 8.70 (1H, s), 0.09 (1H, s);
Mass Spectrum : C₁₅H₁₂SO₂N₂ requires 284; found 285 (MH⁺).

### Description 4

### 8-Iodo-3-phenylsulfonylquinoline (D4)

8-Amino-3-phenylsulfonylquinoline (D3) (31.6 g, 0.11 mol) was dissolved in trifluoroacetic acid (60 ml) and the mixture evaporated. The resulting brown oil was dissolved in acetonitrile (200 ml) and added dropwise to a stirred solution of n-butyl nitrite (6.1 ml) in acetonitrile (300 ml) maintained at a temperature of <5 °C. Once the addition was completed, the mixture was stirred for five minutes then tetra-(n-butyl)ammonium iodide (82 g, 0.22 mol) added portionwise, keeping the temperature below 10 °C. The mixture was stirred for a further 20 minutes then concentrated *in vacuo.* The dark residue was subjected to flash-75 chromatography (2 kg silica gel), eluting with hexane and dichloromethane to give a brown solid. This was dissolved in dichloromethane (500 ml) and washed with 10% aqueous sodium thiosulphate (2 x 300 ml), dried over magnesium sulphate and concentrated to an orange solid. This was triturated with methanol to give the title compound (D4) (25.2 g, 75%) as a light yellow solid;
δ_{H} (CDCl₃) 7.39 (1H, t), 7.53-7.63 (3H, m), 7.96 (1H, d), 8.04 (2H, dd), 8.50 (1H, dd), 8.79 (1H, d), 9.32 (1H, d);
Mass Spectrum : C₁₅H₁₀NO₂SI requires 395; found 396 (MH⁺).

### Description 5

### 3-Phenylsulfonyl-8-piperazin-1-yl-quinoline (D5)

A 100 ml three necked flask was charged with Pd₂(dba)₃ (174 mg, 0.19 mmol, 0.03eq), 8-Iodo-3-phenylsulfonylquinoline (D4) (2.5g, 6.33 mmol), 1,1'-bis-diphenylphosphenoferrocene (316 mg, 0.57 mmol), sodium *tert*butoxide (851 mg, 8.86 mmol, 1.4eq) and piperazine (2.72g, 31.6 mmol, 5eq). The flask was evacuated and filled with nitrogen 4 times then anhydrous 1,4-dioxane (17.5 ml, 7vol) was added. The mixture was stirred and heated to 40°C for 16 ½ hrs.
The dark solution was allowed to cool to room temperature, dichloromethane (12.5 ml) was added and the solution was washed with H₂O (12.5 ml). The aqueous wash was extracted with dichloromethane and the combined organic layers were extracted with 5M HCl (2x12.5 ml). The combined aqueous layers were washed with (dichloromethane 2.5 ml) then transferred to a conical flask, dichloromethane (12.5 ml) was added and the flask was cooled in an ice/water bath. 10M Aqueous sodium hydroxide (13 ml) was added whilst stirring, the mixture was then stirred at room temperature until all the solids were dissolved. The lower organic layer was removed and the aqueous layer was extracted with dichloromethane (7.5 ml), the combined organic layers were concentrated under reduce pressure to ~5 ml. Isooctane (2.5 ml) was added to the dark brown solution resulting in crystallisation, the mixture was stirred at room temp for 5 min then isooctane (22.5 ml) was added over 5 min. The mixture was aged at room temp for 1½ hrs before being cooled in an ice/water bath for 30 min, the mixture was filtered and the cake washed with isooctane (5 ml). The cake was dried under reduced pressure to give the title compound D5; yield 1.67g, 75%.
δ_{H} (CDCl₃): 1.6 (1H, bs), 3.18 (4H, m), 3.34 (4H, m), 7.27 (1H, m), 7.49-7.60 (5H, m), 8.01 (2H, dd), 8.75, (1H, d), 9.21 (1H, d).

### Example 1

### Crystallisation of 3-phenylsulfonyl-8-piperazin-1-yl-quinoline (Form III)

3-Phenylsulfonyl-8-piperazin-1-yl-quinoline (D5) (5g) was dissolved in isopropanol (60ml) with warming to reflux. The resultant solution was then treated with charcoal (1.25g), filtered, then the solution was allowed to cool to ambient, crystallisation initiating at -60°C. The product was collected by filtration, washed with isopropanol (10ml;)and dried *in vacuo* at 45°C to give the title compound, 3.1 g, 62%. Melting point 188°C.

### Example 2

### Crystallisation of 3-phenylsulfonyl-8-piperazin-1-yl-quinoline (Form III)

### (Alternative Procedure)

3-Phenylsulfonyl-8-piperazin-1-yl-quinoline (D5) (10g) was dissolved in ethanol (80ml) with warming to 70°C. The resultant solution was then treated with charcoal (1g), filtered, and the filter bed rinsed with ethanol (20ml). The combined filtrate was adjusted to 54°C, then cooled to 50 °C over 15 minutes. The solution was seeded with 3-phenylsulfonyl-8-piperazin-1-yl-quinoline (Form III) (10mg), cooled to 35°C over 30 minutes, held for 1 hour, then cooled to 20°C over 30 minutes, and stirred for a further 1 hour 15 minutes. The product was collected by filtration, washed with cold ethanol, (2 x 10ml;)and dried *in vacuo* at 40°C to give the title compound, 6.64g, 66.4%. Melting point 188°C.

### Example 3

### Crystallisation of 3-phenylsulfonyl-8-piperazin-1-yl-quinoline (Form III)

### (Alternative Procedure)

3-Phenylsulfonyl-8-piperazin-1-yl-quinoline (D5) (10g) was dissolved in ethanol (80ml) with warming to 72°C. The resultant solution was then pumped through a Cuno R55SP filter into a second vessel (pre-heated to 55°C) *via* a sinter funnel. The original flask and the Cuno filter were washed with ethanol (20ml). The combined filtrate was heated to 72°C to redissolve the material, then adjusted to 50°C. The solution was seeded with 3-phenylsulfonyl-8-piperazin-1-yl-quinoline (Form III) (10mg), cooled to 35°C over 30 minutes, held for 1 hour, then cooled to 20°C over 30 minutes, and stirred for a further 48 hours. The product was collected by filtration, washed with ethanol (20ml) and dried *in vacuo* at 50°C to give the title compound, 5.64g, 56.4%. Melting point 188°C.

### Characterising data recorded for Example 1:

The infrared spectrum of the solid product was recorded using a Nicolet Avatar 360 FT-IR spectrometer fitted with a universal ATR accessory. The FT-IR spectrum (Figure 1) shows bands at: 724, 758, 777, 804, 818, 838, 856, 905, 918, 948, 1023, 1055, 1081, 1092, 1118, 1136, 1153, 1178, 1244, 1302, 1318, 1365, 1378, 1403, 1444, 1471, 1490, 1569, 1584, 1603 and 2819 cm⁻¹.

FT-Raman spectra of samples in glass tubes were acquired using a ThermNicolet 960 E.S.P. spectrometer. Excitation at 1064 nm was provided by a Nd:YVO₄ laser with a power of 400 mW at the sample position. 1200 scans were recorded at 4 cm⁻¹ resolution.
The FT-Raman spectrum (Figure 2) shows bands at 159, 184, 214, 241, 285, 304, 318, 429, 545, 558, 614, 706, 724, 803, 856, 1000, 1023, 1080, 1093, 1136, 1152, 1233, 1243, 1317, 1343, 1364, 1378, 1403, 1446, 1569, 1584, 1602, 3050 and 3073 cm⁻¹.

The X-Ray Powder Diffractogram pattern of the solid product (Figure 3) was recorded using the following acquisition conditions: Unground material was packed into top-filled Si cups. Powder patterns were obtained using a Bruker D8 Advance X-Ray powder diffractometer configured with a Cu anode (40 kV, 40 mA), variable divergence slit, primary and secondary Soller slits, and a position sensitive detector. Data were acquired over the range 2-40 degrees 2-theta using a step size of 0.0145 degrees 2-theta (1 s per step). Samples were rotated during data collection. Characteristic 2θ XRPD angles are 10.29, 10.76, 11.94, 14.33, 14.61, 14.93, 16.02, 16.80, 17.47, 17.92, 19.13, 19.55, 19.84, 20.33, 21.16, 21.36, 23.33, 23.96, 24.44, 24.67, 25.51, 26.12, 27.13, 27.77, 28.06, 28.35, 29.23, 29.46, 30.06, 30.35, 31.27, 32.35, 32.66, 33.08, 33.77, 34.49, 35.18, 36.42, 37.34, 38.39 and 39.51°.

### Brief Description of the Drawings

Figure 1 shows the Infrared spectrum obtained for 3-phenylsulfonyl-8-piperazin-1-yl-quinoline (Form III).
Figure 2 shows the Raman spectrum obtained for 3-phenylsulfonyl-8-piperazin-1-yl-quinoline (Form III).
Figure 3 shows the X-Ray Powder Diffractogram obtained for 3-phenylsulfonyl-8-piperazin-1-yl-quinoline (Form III).

## Claims

1. A polymorphic form of 3-phenylsulfonyl-8-piperazin-1-yl-quinoline **characterised in that** it provides:
an infrared spectrum containing peaks at 724, 758, 777, 804, 818, 838, 856, 905, 918, 948, 1023, 1055, 1081, 1092, 1118, 1136, 1153, 1178, 1244, 1302, 1318, 1365, 1378, 1403, 1444, 1471, 1490, 1569, 1584, 1603 and 2819 cm⁻¹.

2. A polymorphic form of 3-phenylsulfonyl-8-piperazin-1-yl-quinoline **characterised in that** it provides:
a Raman spectrum containing peaks at 159, 184, 214, 241, 285, 304, 318, 429, 545, 558, 614, 706, 724, 803, 856, 1000, 1023, 1080, 1093, 1136, 1152, 1233, 1243, 1317, 1343, 1364, 1378, 1403, 1446, 1569, 1584, 1602, 3050 and 3073 cm⁻¹.

3. A polymorphic form of 3-phenylsulfonyl-8-piperazin-1-yl-quinoline **characterised in that** it provides:
an X-ray powder diffraction (XRPD) pattern which gives calculated lattice spacings at 10.29, 11.94, 17.47, 19.55, 19.84, and 20.33°.

4. A polymorphic form of 3-phenylsulfonyl-8-piperazin-1-yl-quinoline **characterised in that** it provides:
a melting point of 188°C.

5. A polymorph according to claim 1 which provides an infrared spectrum substantially in accordance with Figure 1.

6. A polymorph according to claim 2 which provides a Raman spectrum substantially in accordance with Figure 2.

7. A polymorph according to claim 3 which provides an X-ray powder diffraction (XRPD) pattern which gives calculated lattice spacings at 10.29, 10.76, 11.94, 14.33, 14.61, 14.93, 16.02, 16.80, 17.47, 17.92, 19.13, 19.55, 19.84, 20.33, 21.16, 21.36, 23.33, 23.96, 24.44, 24.67, 25.51, 26.12, 27.13, 27.77, 28.06, 28.35, 29.23, 29.46, 30.06, 30.35, 31.27, 32.35, 32.66, 33.08, 33.77, 34.49, 35.18, 36.42, 37.34, 38.39 and 39.51°.

8. A polymorph according to any one of claims 3 or 7 which provides an X-ray powder diffraction (XRPD) pattern substantially in accordance with Figure 3.

9. A polymorph according to any one of claims 1 to 8, in isolated form.

10. A polymorph according to any one of claims 1 to 8, in pure form.

11. A polymorph according to any one of claims 1 to 8 in an amount greater than 90% relative to other forms of 3-phenylsulfonyl-8-piperazin-1yl-quinoline.

12. A polymorph according to any one of claims 1 to 8, in crystalline form.

13. A pharmaceutical composition which comprises a polymorph according to any one of claims 1 to 12 and a pharmaceutically acceptable carrier or excipient.

14. A polymorph according to any one of claims 1 to 12 for use in therapy.

15. A polymorph according to any one of claims 1 to 12 for use in the treatment of anxiety, depression, epilepsy, obsessive compulsive disorders, migraine, cognitive memory disorders, Parkinsons Disease, ADHD, sleep disorders, feeding disorders such as anorexia and bulimia, panic attacks, withdrawal from drug abuse such as cocaine, ethanol, nicotine and benzodiazepines, schizophrenia, stroke and also disorders associated with spinal trauma and/or head injury, IBS or obesity.

16. Use of a polymorph according to any one of claims 1 to 12 in the manufacture of a medicament for the treatment or prophylaxis of anxiety, depression, epilepsy, obsessive compulsive disorders, migraine, cognitive memory disorders, Parkinsons Disease, ADHD, sleep disorders, feeding disorders such as anorexia and bulimia, panic attacks, withdrawal from drug abuse such as cocaine, ethanol, nicotine and benzodiazepines, schizophrenia, stroke and also disorders associated with spinal trauma and/or head injury, IBS or obesity.

17. A pharmaceutical composition comprising a polymorph according to any one of claims 1 to 12 for use in the treatment of anxiety, depression, epilepsy, obsessive compulsive disorders, migraine, cognitive memory disorders, Parkinsons Disease, ADHD, sleep disorders, feeding disorders such as anorexia and bulimia, panic attacks, withdrawal from drug abuse such as cocaine, ethanol, nicotine and benzodiazepines, schizophrenia, stroke and also disorders associated with spinal trauma and/or head injury, IBS or obesity.

18. A polymorph, a use or a pharmaceutical compostion according to any one of claims 15 to 17 wherein the cognitive memory disorders are Alzheimers disease, age related cognitive decline and mild cognitive impairment.

19. A polymorph, a use or a pharmaceutical compostion according to any one of claims 15 to 17 wherein the treatment is for depression, anxiety, Alzheimers disease, age related cognitive decline, ADHD, obesity, mild cognitive impairment, schizophrenia, cognitive deficits in schizophrenia and stroke.

20. Use of a polymorph according to any one of claims 1 to 12 in the manufacture of a medicament for promoting neuronal growth within the central nervous system of a mammal.

## Patentansprüche

1. Polymorphe Form von 3-Phenylsulfonyl-8-piperazin-1-yl-chinolin, **dadurch gekennzeichnet, dass** sie ein Infrarotspektrum enthaltend Peaks bei 724, 758, 777, 804, 818, 838, 856, 905, 918, 948, 1023, 1055, 1081, 1092, 1118, 1136, 1153, 1178, 1244, 1302, 1318, 1365, 1378, 1403, 1444, 1471, 1490, 1569, 1584, 1603 und 2819 cm⁻¹ aufweist.

2. Polymorphe Form von 3-Phenylsulfonyl-8-piperazin-1-yl-chinolin, **dadurch gekennzeichnet, dass** sie ein Raman-Spektrum enthaltend Peaks bei 159, 184, 214, 241, 285, 304, 318, 429, 545, 558, 614, 706, 724, 803, 856, 1000, 1023, 1080, 1093, 1136, 1152, 1233, 1243, 1317, 1343, 1364, 1378, 1403, 1446, 1569, 1584, 1602, 3050 und 3073 cm⁻¹ aufweist.

3. Polymorphe Form von 3-Phenylsulfonyl-8-piperazin-1-yl-chinolin, **dadurch gekennzeichnet, dass** sie ein Röntgenpulverbeugungsdiagramm (XRPD) aufweist, welches berechnete Gitterabstände bei 10,29, 11,94, 17,47, 19,55, 19,84 und 20,33° aufweist.

4. Polymorphe Form von 3-Phenylsulfonyl-8-piperazin-1-yl-chinolin, **dadurch gekennzeichnet, dass** sie einen Schmelzpunkt von 188 °C aufweist.

5. Polymorph gemäß Anspruch 1, welches ein Infrarotspektrum im Wesentlichen in Übereinstimmung mit Figur 1 aufweist.

6. Polymorph gemäß Anspruch 2, welches ein Raman-Spektrum im Wesentlichen in Übereinstimmung mit Figur 2 aufweist.

7. Polymorph gemäß Anspruch 3, welches ein Röntgenpulverbeugungsdiagramm (XRPD) aufweist, das berechnete Gitterabstände bei 10,29, 10,76, 11,94, 14,33, 14,61, 14,93, 16,02, 16,80, 17,47, 17,92, 19,13, 19,55, 19,84, 20,33, 21,16, 21,36, 23,33, 23,96, 24,44, 24,67, 25,51, 26,12, 27,13, 27,77, 28,06, 28,35, 29,23, 29,46, 30,06, 30,35, 31,27, 32,35, 32,66, 33,08, 33,77, 34,49, 35,18, 36,42, 37,34, 38,39 und 39,51° aufweist.

8. Polymorph gemäß Anspruch 3 oder 7, welches ein Röntgenpulverbeugungsdiagramm (XRPD) im Wesentlichen in Übereinstimmung mit Figur 3 aufweist.

9. Polymorph gemäß einem der Ansprüche 1 bis 8 in isolierter Form.

10. Polymorph gemäß einem der Ansprüche 1 bis 8 in reiner Form.

11. Polymorph gemäß einem der Ansprüche 1 bis 8, das in einer Menge von mehr als 90% im Vergleich zu anderen Formen von 3-Phenylsulfonyl-8-piperazin-1-yl-chinolin vorliegt.

12. Polymorph gemäß einem der Ansprüche 1 bis 11 in kristalliner Form.

13. Arzneimittel, umfassend ein Polymorph gemäß einem der Ansprüche 1 bis 12 und einen pharmazeutisch verträglichen Träger oder Exzipienten.

14. Polymorph gemäß einem der Ansprüche 1 bis 12 zur Verwendung in der Therapie.

15. Polymorph gemäß einem der Ansprüche 1 bis 12 für die Verwendung zur Behandlung von Angstzuständen, Depression, Epilepsie, Zwangsstörungen, Migräne, kognitiven Gedächtnisstörungen, Parkinson'scher Krankheit, ADHD, Schlafstörungen, Esstörungen wie Anorexie und Bulimie, Panikattacken, Entzug vom Drogenmissbrauch wie Kokain, Ethanol, Nikotin und Benzodiazepinen, Schizophrenie, Schlaganfall und auch Störungen, die mit Spinaltrauma und/oder Kopfverletzung in Zusammenhang stehen, IBS oder Fettsucht.

16. Verwendung eines Polymorphs gemäß einem der Ansprüche 1 bis 12 für die Herstellung eines Arzneimittels zur Behandlung oder Prophylaxe von Angstzuständen, Depression, Epilepsie, Zwangsstörungen, Migräne, kognitiven Gedächtnisstörungen, Parkinson'scher Krankheit, ADHD, Schlafstörungen, Esstörungen wie Anorexie und Bulimie, Panikattacken, Entzug vom Drogenmissbrauch wie Kokain, Ethanol, Nikotin und Benzodiazepinen, Schizophrenie, Schlaganfall und auch Störungen, die mit Spinaltrauma und/oder Kopfverletzung in Zusammenhang stehen, IBS oder Fettsucht.

17. Arzneimittel umfassend ein Polymorph gemäß einem der Ansprüche 1 bis 12 für die Verwendung zur Behandlung von Angstzuständen, Depression, Epilepsie, Zwangsstörungen, Migräne, kognitiven Gedächtnisstörungen, Parkinson'scher Krankheit, ADHD, Schlafstörungen, Esstörungen wie Anorexie und Bulimie, Panikattacken, Entzug vom Drogenmissbrauch wie Kokain, Ethanol, Nikotin und Benzodiazepinen, Schizophrenie, Schlaganfall und auch Störungen, die mit Spinaltrauma und/oder Kopfverletzung in Zusammenhang stehen, IBS oder Fettsucht.

18. Polymorph, Verwendung oder Arzneimittel gemäß einem der Ansprüche 15 bis 17, wobei die kognitiven Gedächtnisstörungen Alzheimer'sche Krankheit, altersbedingter Abbau kognitiver Fähigkeiten und leichte kognitive Störung sind.

19. Polymorph, Verwendung oder Arzneimittel gemäß einem der Ansprüche 15 bis 17, wobei die Behandlung gegen Depression, Angstzustände, Alzheimer'sche Krankheit, altersbedingten Abbau kognitiver Fähigkeiten, ADHD, Fettsucht, leichte kognitive Störung, Schizophrenie, kognitive Defizite bei Schizophrenie und Schlaganfall ist.

20. Verwendung eines Polymorphs gemäß einem der Ansprüche 1 bis 12 für die Herstellung eines Arzneimittels zur Förderung von Neuronenwachstum im zentralen Nervensystem einer Säugers.

## Revendications

1. Forme polymorphe de 3-phénylsulfonyl-8-pipérazine-1-yl-quinoléine, **caractérisée en ce qu'**elle présente :
un spectre infrarouge contenant des pics à 724, 758, 777, 804, 818, 838, 856, 905, 918, 948, 1023, 1055, 1081, 1092, 1118, 1136, 1153, 1178, 1244, 1302, 1318, 1365, 1378, 1403, 1444, 1471, 1490, 1569, 1584, 1603 et 2819 cm⁻¹.

2. Forme polymorphe de 3-phénylsulfonyl-8-pipérazine-1-yl-quinoléine, **caractérisée en ce qu'**elle présente :
un spectre Raman contenant des pics à 159, 184, 214, 241, 285, 304, 318, 429, 545, 558, 614, 706, 724, 803, 856, 1000, 1023, 1080, 1093, 1136, 1152, 1233, 1243, 1317, 1343, 1364, 1378, 1403, 1446, 1569, 1584, 1602, 3050 et 3073 cm⁻¹.

3. Forme polymorphe de 3-phénylsulfonyl-8-pipérazine-1-yl-quinoléine, **caractérisée en ce qu'**elle présente :
un diagramme de diffraction des rayons X sur poudre (XRPD) qui donne des distances de réseau calculées à 10,29, 11,94, 17, 47, 19,55, 19,84 et 20,33°.

4. Forme polymorphe de 3-phénylsulfonyl-8-pipérazine-1-yl-quinoléine, **caractérisée en ce qu'**elle présente :
un point de fusion de 188°C.

5. Forme polymorphe suivant la revendication 1, qui présente un spectre infrarouge substantiellement suivant la figure 1.

6. Forme polymorphe suivant la revendication 2, qui présente un spectre Raman substantiellement suivant la figure 2.

7. Forme polymorphe suivant la revendication 3, qui présente un diagramme de diffraction des rayons X sur poudre (XRPD) qui donne, des distances de réseau calculées à 10,29, 10,76, 11, 94, 14,33, 14,61, 14,93, 16,02, 16,80, 17,47, 17,92, 19,13, 19,55, 19,84, 20,33, 21,16, 21,36, 23,33, 23,96, 24,44, 24,67, 25,51, 26,12, 27,13, 27,77, 28,06, 28,35, 29,23, 29,46, 30,06, 30,35, 31,27, 32,35, 32,66, 33,08, 33,77, 34,49, 35,18, 36,12, 37,34, 38,39 et 39,51°.

8. Forme polymorphe suivant l'une quelconque des revendications 3 ou 7, qui présente un diagramme de diffraction des rayons X sur poudre (XRPD) substantiellement suivant la figure 3.

9. Forme polymorphe suivant l'une quelconque des revendications 1 à 8, sous forme isolée.

10. Forme polymorphe suivant l'une quelconque des revendications 1 à 8, sous forme pure.

11. Forme polymorphe suivant l'une quelconque des revendications 1 à 8, présente en une quantité supérieure à 90 % par rapport aux autres formes de 3-phénylsulfonyl-8-pipérazine-1-yl-quinoléine.

12. Forme polymorphe suivant l'une quelconque des revendications 1 à 11, sous forme cristalline.

13. Composition pharmaceutique qui comprend une forme polymorphe suivant l'une quelconque des revendications 1 à 12 et un support ou excipient pharmaceutiquement acceptable.

14. Forme polymorphe suivant l'une quelconque des revendications 1 à 12, destinée à être utilisée en thérapie.

15. Forme polymorphe suivant l'une quelconque des revendications 1 à 12, destinée à être utilisée dans le traitement de l'anxiété, de la dépression, de l'épilepsie, de troubles obsessionnels compulsifs, de la migraine, de troubles cognitifs de la mémoire, de la maladie de Parkinson, de l'ADHD, de troubles du sommeil, de troubles de l'alimentation tels que l'anorexie et la boulimie, d'attaques de panique, du syndrome de privation après l'abus de substances médicamenteuses telles que la cocaïne, l'éthanol, la nicotine et les benzodiazépines, de la schizophrénie, d'un ictus et également de troubles associés à un traumatisme rachidien et/ou une lésion crânienne, du IBS ou de l'obésité.

16. Utilisation d'une forme polymorphe suivant l'une quelconque des revendications 1 à 12 dans la production d'un médicament destiné au traitement ou à la prophylaxie de l'anxiété, de la dépression, de l'épilepsie, de troubles obsessionnels compulsifs, de la migraine, de troubles cognitifs de la mémoire, de la maladie de Parkinson, de l'ADHD, de troubles du sommeil, de troubles de l'alimentation tels que l'anorexie et la boulimie, d'attaques de panique, du syndrome de privation après l'abus de substances médicamenteuses telles que la cocaïne, l'éthanol, la nicotine et les benzodiazépines, de la schizophrénie, d'un ictus et également de troubles associés à un traumatisme rachidien et/ou une lésion crânienne, du IBS ou de l'obésité.

17. Composition pharmaceutique comprenant une forme polymorphe suivant l'une quelconque des revendications 1 à 12, destinée à être utilisée dans le traitement de l'anxiété, de la dépression, de l'épilepsie, de troubles obsessionnels compulsifs, de la migraine, de troubles cognitifs de la mémoire, de la maladie de Parkinson, de l'ADHD, de troubles du sommeil, de troubles de l'alimentation tels que l'anorexie et la boulimie, d'attaques de panique, du syndrome de privation après l'abus de substances médicamenteuses telles que la cocaïne, l'éthanol, la nicotine et les benzodiazépines, de la schizophrénie, d'un ictus et également de troubles associés à un traumatisme rachidien et/ou une lésion crânienne, du IBS ou de l'obésité.

18. Forme polymorphe, utilisation ou composition pharmaceutique suivant l'une quelconque des revendications 15 à 17, dans laquelle les troubles cognitifs de la mémoire sont la maladie d'Alzheimer, le déclin cognitif dû à l'âge et l'altération cognitive faible bénigne.

19. Forme polymorphe, utilisation ou composition pharmaceutique suivant l'une quelconque des revendications 15 à 17, dans laquelle le traitement est destiné à la dépression, l'anxiété, la maladie d'Alzheimer, le déclin cognitif dû à l'âge, l'ADHD, l'obésité, une altération cognitive bénigne, la schizophrénie, des déficits cognitifs dans la schizophrénie et un ictus.

20. Utilisation d'une forme polymorphe suivant l'une quelconque des revendications 1 à 12 dans la production d'un médicament destiné à favoriser la croissance neuronale dans le système nerveux central d'un mammifère.
